(19) Europäisches Patentamt
European Patent Office
Office européen des brevets

(11) Veröffentlichungsnummer: **0 214 565 A1**

# (12) EUROPÄISCHE PATENTANMELDUNG

(21) Anmeldenummer: 86111947.7

(22) Anmeldetag: 29.08.86

(51) Int. Cl.[4]: **C07C 49/813** , C07C 45/63

(30) Priorität: 06.09.85 DE 3531838

(43) Veröffentlichungstag der Anmeldung: 18.03.87 Patentblatt 87/12

(84) Benannte Vertragsstaaten: CH DE FR GB IT LI

(71) Anmelder: **BASF Aktiengesellschaft**
**Carl-Bosch-Strasse 38**
**D-6700 Ludwigshafen(DE)**

(72) Erfinder: **Neumann, Peter, Dr.**
**Franz-Schubert-Strasse 1**
**D-6908 Wiesloch(DE)**
Erfinder: **Schaefer, Gerhard, Dr.**
**Schafrippel 2**
**D-6900 Heidelberg(DE)**

## (54) Verfahren zur Herstellung von Bis-(fluorbenzoyl)-Verbindungen.

(57) Bis(fluorbenzoyl)-Verbindungen werden hergestellt durch Umsetzung einer Verbindung der Formel

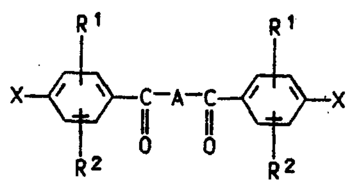

in der X Brom oder Chlor bedeutet, mit einem anorganischen Salz der Fluorwasserstoffsäure.

EP 0 214 565 A1

## Verfahren zur Herstellung von Bis-(fluorbenzoyl)-Verbindungen

Aromatische Fluorketone können durch Friedel-Crafts-Acylierung von Fluoraromaten hergestellt werden. Die bei diesem Verfahren eingesetzten Fluoraromaten sind jedoch häufig nur mit hohem Aufwand herstellbar und daher teuer.

Der Erfindung lag nun die Aufgabe zugrunde, ein einfaches und wirtschaftliches Verfahren zur Herstellung von Bis(fluorbenzoyl)-Verbindungen zu entwickeln.

Gegenstand der Erfindung ist ein Verfahren zur Herstellung von Bis-fluorbenzoylverbindungen der Formel

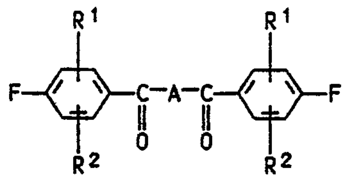

(I) ,

in der die Symbole folgende Bedeutung haben:

$R^1$ und $R^2$, die gleich oder verschieden sein können, sind Wasserstoff, Methyl, Ethyl, Methoxy oder Ethoxy,

A ist ein ein-oder mehrkerniger carbocyclischer oder heterocyclischer aromatischer Rest oder einen Rest der Formel

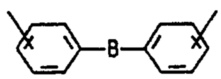

,

in der B für eine chemische Bindung, O, S, SO, $SO_2$, $(CH_2)_n$ (wobei n eine ganze Zahl von 1 bis 4 ist), $C(CH_3)_2$, CO oder $Si(CH_3)_2$, steht, wobei man eine Verbindung der Formel

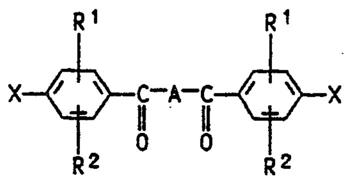

(II),

in der X für ein Brom-oder vorzugsweise Chloratom steht, und $R^1$, $R^2$ und A die oben genannte Bedeutung haben, mit einem anorganischen Salz der Fluorwasserstoffsäure, gegebenenfalls in Gegenwart eines Lösungsmittels, bei Temperaturen von 100 bis 500°C, vorzugsweise 350 bis 450°C, umsetzt.

Die als Ausgangsstoffe eingesetzten Verbindungen der Formel II werden auf übliche Weise hergestellt durch Friedel-Crafts-Acylierung der gut zugänglichen Brom-oder vorzugsweise Chlorbenzolderivate der Formel

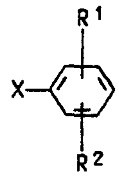

(III),

in der X, $R^1$ und $R^2$ die oben genannte Bedeutung haben, mit Dicarbonsäurechloriden der Formel

Y-CO-A-CO-Y (IV),

in der Y für ein Fluor, Brom oder vorzugsweise Chloratom steht und A die oben genannte Bedeutung hat.

Als anorganische Salze der Fluorwasserstoffsäure kommen insbesondere Alkalifluoride, wie Lithium-, Natrium-, Kalium-, Rubidium-oder Caesiumfluorid, vor allem Kaliumfluorid, gegebenenfalls in Mischung Caesiumfluorid, in Frage.

Bei der Durchführung der Reaktion in der Schmelze wird die Bis-chlorbenzoylverbindung der Formel II vorzugsweise mit überschüssigem Kaliumfluorid (10 bis 100 % Mol.% Überschuß, bezogen auf austauschbares Chlor-oder Brom) 2 Stunden auf 380 bis 420°C erhitzt. Zur Reinigung wird das Reaktionsgemisch nach Abkühlen mit einem Lösungsmittel, z.B. Chlorbenzol versetzt, aufgekocht, der Rückstand abfiltriert, und der aus der Mutterlauge beim Abkühlen auskristallisierende Niederschlag in üblicher Weise durch Absaugen isoliert.

Die Umsetzung kann unter gleichen Bedingungen auch in Gegenwart eines Lösungsmittels durchgeführt werden.

Als Lösungsmittel sind beispielsweise Chlorbenzol, Dichlorbenzol, Trichlorbenzol, Dimethylsulfon, Diphenylsulfon, Dimethylsulfoxid, Sulfolan, N,N'-Dimethylethylenharnstoff, N,N'-Dimethylpropylenharnstoff oder N-Methylpyrrolidon zu nennen.

Die Umsetzung kann drucklos oder unter Druck durchgeführt werden, bei niedrig siedenden Lösungsmitteln ist Druckfahrweise vorteilhaft.

Zur Aufarbeitung wird das Lösungsmittel unter vermindertem Druck abdestilliert und das Rohprodukt in der oben beschriebenen Weise gereinigt.

Man kann die unlöslichen Rückstände aus der Reaktionsmischung auch durch Filtration, gegebenenfalls bei erhöhter Temperatur entfernen und das Reaktionsprodukt direkt aus dem Filtrat kristallisieren lassen oder nach Entfernen des Lösungsmittels, wie oben beschrieben, isolieren.

Als Endprodukt des erfindungsgemäßen Verfahrens erhält man Bis-(fluorbenzoyl)-Verbindungen der Formel

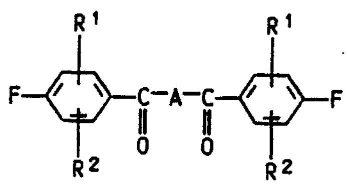

(I),

in der $R^1$, $R^2$ und A die oben genannte Bedeutung haben. Als besonders bevorzugte Verbindungen der Formel I sind zu nennen:

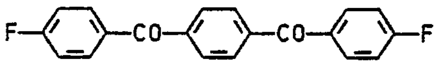 , 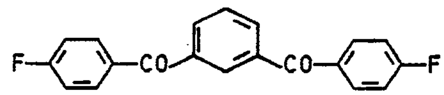 ,

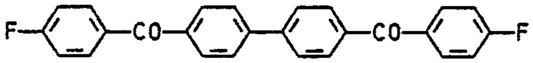 , 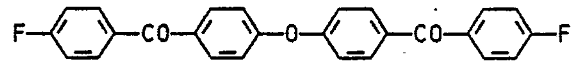 ,

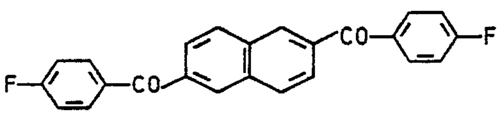 .

Die erfindungsgemäß hergestellten Fluorbenzoylverbindungen eignen sich als Ausgangsstoffe für die Synthese von Polymeren, insbesondere von Polyetherketonen.

Die Erfindung wird durch die nachstehenden Beispiele näher erläutert.

Beispiel 1

Eine Mischung aus 177,5 g 1,4-Bis(4-chlorbenzoyl)benzol und 116 g wasserfreiem Kalziumfluorid wird innerhalb von 2 Stunden auf 400°C erhitzt. Man hält das Reaktionsprodukt noch 2 Stunden bei 380 bis 420°C, nach dem Abkühlen gibt man Chlorbenzol zu, kocht auf, filtriert heiß und saugt den nach dem Erkalten ausgefallenen Niederschlag ab, wäscht mit Methanol und trocknet. Man erhält 130 g (81 % d.Th.) 1,4-Bis(4-fluorbenzoyl)benzol vom Schmp. 218°C.

Beispiel 2

Eine Mischung aus 355 g 1,4-Bis(4-chlorbenzoyl)benzol, 232 g wasserfreiem Kaliumfluorid und 218 g Diphenylsulfon wird innerhalb von 2,5 Stunden auf 400°C erwärmt und noch 3 Stunden bei 380 bis 420°C gerührt. Anschließend destilliert man das Lösungsmittel unter vermindertem Druck ab und behandelt den Rückstand, wie in Beispiel 1 beschrieben, mit Chlorbenzol. Man erhält 251 g 1,4-Bis(fluorbenzoyl)benzol vom Schmp. 218 bis 219°C.

## Ansprüche

1. Verfahren zur Herstellung von Bis-(fluorbenzoyl)-Verbindungen der Formel

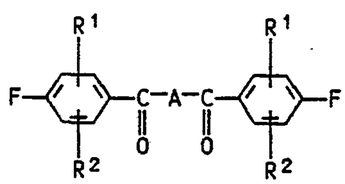 ,

in der die Symbole folgende Bedeutung haben:

$R^1$ und $R^2$, die gleich oder verschieden sein können, sind Wasserstoff, Methyl, Ethyl, Methoxy oder Ethoxy

A ist ein ein-oder mehrkerniger carbocyclischer oder heterocyclischer aromatischer Rest oder ein Rest der Formel

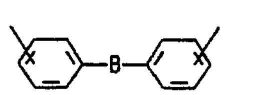 ,

in der B für eine chemische Bindung, O, S, SO, $SO_2$, $(CH_2)_n$ (wobei n eine ganze Zahl von 1 bis 4 ist), $C(CH_3)_2$, CO oder $Si(CH_3)_2$, steht, dadurch gekennzeichnet, daß man eine Verbindung der Formel

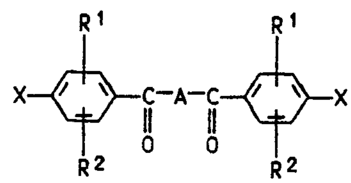

in der X für ein Brom-oder vorzugsweise CHloratom steht, und R', $R^2$ und A die oben genannte Bedeutung haben, mit einem anorganischen Salz der Fluorwasserstoffsäure bei Temperaturen von 100 bis 500°C versetzt.

2. Verfahren nach Anspruch 1, dadurch gekennzeichnet, daß man 1,4-Bis-(chlorbenzoyl)-benzol mit Kaliumfluorid umsetzt.

Europäisches Patentamt

# EUROPÄISCHER RECHERCHENBERICHT

Nummer der Anmeldung

EP 86111947.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 101 760 (MITSUI TDATSU CHEMICALS)<br>* Ansprüche *<br>-- | 1,2 | C 07 C 49/813<br>C 07 C 45/63 |
| A | DE - A1 - 2 810 794 (IMPERIAL CHEMICAL INDUSTRIES)<br>* Ansprüche 1,5,6; Seite 9, Zeile 19 *<br>-- | 1 | |
| A | DE - A - 2 425 199 (IMPERIAL CHEMICAL INDUSTRIES)<br>* Ansprüche *<br>---- | 1 | |

RECHERCHIERTE SACHGEBIETE (Int. Cl.4)

C 07 C 49/00
C 07 C 45/00

Der vorliegende Recherchenbericht wurde für alle Patentansprüche erstellt.

| Recherchenort | Abschlußdatum der Recherche | Prüfer |
|---|---|---|
| WIEN | 07-11-1986 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82